# EUROPEAN PATENT SPECIFICATION

(11) **EP 1 874 300 B1**
(45) Date of publication and mention of the grant of the patent: **03.10.2012**
(21) Application number: 06758559.6
(22) Date of filing: 19.04.2006
(51) Int. Cl.: A61K 31/4015, A61K 31/454, A61P 25/00, A61P 25/28

(54) **Method for treating apathy syndrome**
Verfahren zur Behandlung des Apathie-Syndroms
Procédé de traitement du syndrome d'apathie

(30) Priority: 17.04.2006 US 406158; 20.04.2005 US 673555 P
(43) Date of publication of application: 09.01.2008
(73) Proprietor: Hamilton Pharmaceuticals, Inc., Washington, DC 20006 (US)
(72) Inventor: CHA, Albert, Palo Alto, California 94301 (US)
(74) Representative: den Hartog, Jeroen H.J.
(86) International application number: PCT/US2006/015513
(87) International publication number: WO 2006/113937

(56) References cited:
- WO-A1-03/018005
- JP-A- 9 087 178
- TACKE B ET AL: "[Pilot study of 2-pyrrolidon-acetamid (generic name: piracetam" PHARMAPSYCHIATRIE, NEURO-PSYCHOPHARMAKOLOGIE, THIEME VERLAG, STUTTGART, DE, vol. 8, no. 2, 1 March 1975 (1975-03-01), pages 82-89, XP009137882 ISSN: 0031-7098
- TREVISIO A ET AL: "Piracetam in the treatment of language disturbances during development" MINERVA PEDIATRICA, TORINO, IT, vol. 29, no. 19, 1 January 1977 (1977-01-01), pages 1267-1272, XP009137884 ISSN: 0026-4946
- OKADA K ET AL: "Poststroke apathy and regional cerebral blood flow" STROKE, LIPPINCOTT WILLIAMS & WILKINS, US, vol. 28, no. 12, 1 December 1997 (1997-12-01), pages 2437-2441, XP009137892 ISSN: 0039-2499
- GLENN MEL B ET AL: "Cutoff score on the apathy evaluation scale in subjects with traumatic brain injury" BRAIN INJURY, TAYLOR AND FRANCIS, LONDON, GB, vol. 16, no. 6, 1 June 2002 (2002-06-01), pages 509-516, XP009137878 ISSN: 0269-9052
- KALECHSTEIN ARI D ET AL: "Apathy syndrome in cocaine dependence" PSYCHIATRY RESEARCH, ELSEVIER IRELAND LTD, IE, vol. 109, no. 1, 31 January 2002 (2002-01-31), pages 97-100, XP009137874 ISSN: 0165-1781
- ROBINSON ROBERT G ET AL: "Double-blind treatment of apathy in patients with poststroke depression using nefiracetam" JOURNAL OF NEUROPSYCHIATRY AND CLINICAL NEUROSCIENCE, AMERICAN PSYCHIATRIC PRESS, WASHINGTON, DC, US, vol. 21, no. 2, 21 March 2009 (2009-03-21) , pages 144-151, XP009137883 ISSN: 0895-0172
- ROBERT P.H. ET AL.: 'The Apathy Inventory: assessment of apathy and awareness in Alzheimer's disease, Parkinson's disease and mild cognitive impairment' INT. J. GERIATR. PSYCHIATRY vol. 17, 2002, pages 1099 - 1105, XP003013783
- NARAHASHI T. ET AL.: 'Unique mechanism of action of Alzheimer's drugs on brain nicotinic acetylcholine receptors and NMDA receptors' LIFE SCIENCES vol. 74, 2003, pages 281 - 291, XP003013784

## Description

### FIELD OF THE INVENTION

The present invention concerns the use of nefiracetam compound in the treatment of apathy syndrome.

### BACKGROUND OF THE INVENTION

Apathy syndrome is a loss of motivation often attributable to a CNS disorder, often involving frontal-subcortical system dysfunction (Van Reekum, et al., J. Neuropsychi. Clin Neurosci., 17 (1): 7-19, 2005). The terms "apathy" and "apathy syndrome" as used herein are not to be confused with apathy as a symptom, which is defined a loss of motivation attributable to emotional distress or diminished level of consciousness. The syndrome is primarily characterized by a diminution in goal-directed overt behaviors. In the differential diagnosis of apathy syndrome (hereinafter "apathy"), akinesia and akinetic mutism, depression, dementia, delirium, despair, and demoralization must be ruled out. Apathy is also significantly associated with older age and cognitive impairment. Although major depression can be associated with an increased frequency of apathy, depression and apathy can exist independent of one another (Marin, et al. J. Nerv. Ment. Dis. 1994 182(4):235-9 (1994)). Apathy is significantly associated with specific cognitive impairments, and can have a different mechanism than depression (Starkstein, et al., J. Neuropsychiatry Clin. Neurosciences, 4: 134-139 (1992)).

Apathy has been linked to a variety of adverse outcomes. For example, apathy is associated with functional impairment among patients and elevated stress among their caregivers. As a consequence, hospitalization is prolonged, therapeutic responses diminish, and institutionalization occurs earlier. Apathy represents a form of executive cognitive dysfunction. Over the past decade, validated criteria and rating scales for apathy have been published. Two of these, described below, are the Apathy Evaluation Scale (AES) and the less comprehensive Apathy Scale.

As indicated above, apathy is common across a number of CNS disorders, often those associated with or characterized by frontal-subcortical system dysfunction. These include but are not limited to stroke, Alzheimer's disease (Landes), Pick's disease, Binswanger's encephalopathy, Parkinson's disease, Huntington's disease, traumatic brain injury, Wernicke-Korsakoff syndrome, Kluver-Bucy syndrome, hydrocephatus, pituitary adenoma and other brain tumors, major depressive disorder, schizophrenia, multiple sclerosis, carbon monoxide poisoning, and chronic cerebral (Lyme disease, hepatitis C, HIV) infection (Duffy). In addition, apathy has been described secondary to various medical disorders (hypothyroidism, pseudohypoparathyroidism, and testosterone deficiency) and drug usage (neuroleptics, selective serotonin uptake inhibitors, marijuana). Combined, it has been estimated that apathy afflicts over 10 million Americans.

Apathy syndrome frequently occurs in stroke patients and can be accompanied by cognitive impairment, depressive state, or other signs of frontal lobe dysfunction. Hypoactivity in the frontal lobe and anterior temporal regions can contribute to the clinical appearance of apathy after stroke. Apathy also affects over 70% of individuals with Alzheimer's disease (AD) and is the most common neuropsychiatric symptom reported in AD patients, but is under-recognized.

Apathy in Parkinson's disease can be distinguished from other psychiatric symptoms and personality features that are associated with the disease, and it is often associated with cognitive impairment. Apathy is one of the major negative symptoms of schizophrenia, which is among the world's top ten causes of long-term disability. Symptoms of schizophrenia include psychosis, apathy and withdrawal, and cognitive impairment, which lead to problems in social and occupational functioning as well as self-care. Apathy is estimated to affect up to 90 percent of schizophrenic patients. Apathy is also frequently associated with major depressive episodes. Depending on the type and severity of comorbid condition, apathy can occur in as many as 86% of patients with depression.

Apathy is one of the primary neuropsychiatric manifestations of frontal system dysfunction and has been associated with neuropathological changes in frontal brain regions. Indeed, frontal-subcortical system dysfunction has been implicated in the causation of apathy; apathy subtypes based on the various frontal-subcortical loops can thus exist. As noted above, both focal lesions (e.g., stroke or traumatic injury) and diffuse neurodegenerative processes (e.g., Alzheimer and Parkinson disease) can produce apathy and other personality changes based on the interruption of projections within or between prefrontal cortex or subcortical structures that comprise the frontal-subcortical circuits. Functional disorders, such as schizophrenia, involving frontal-subcortical neurocircuitry in association with characteristic changes in specific neurotransmitters can also give rise to apathy.

Although apathy can occur in association with disorders where frontal-subcortical system dysfunction has not been implicated, frontal-subcortical circuits provide a comprehensive framework for understanding the anatomy, biochemistry, and pharmacology of apathy and certain other human behaviors. The three principal behaviorally relevant circuits originate in the dorsolateral prefrontal cortex, orbitofrontal cortex, and anterior cingulate cortex, respectively. System-specific marker behaviors associated with each circuit are executive dysfunction (dorsolateral prefrontal-subcortical circuit), disinhibition and OCD (orbitofrontal-subcoztical circuits), and apathy (medial frontal-subcortical circuit). The actions of PCP, LSD, serotonergio antidepressants, anxiolytics, sedative-hypnotics, antipsychotic agents, and ethanol can all be partially or primarily mediated through transmitter systems and receptor effects expressed through frontal-subcortical circuits.

Recent research has identified neurotransmitters mediating synaptic transmission in the frontal-subcortical pathways. These transmitters include acetylcholine and monoamines, especially serotonin, dopamine and norepinephrine. Drugs affecting one or more of these transmitter systems might thus be linked to the clinical appearance of apathy. Conversely, pharmaceutical agents that benefit apathy might be expected to affect these systems.

At present, there is no FDA approved medication for apathy. There is also no proof of efficacy for any drug in current (off-label) use. Some preliminary results suggest that treatment efficacy can exist for some dopaminergic drugs and amphetamines (Czernecki, et al., Neuropsychologia, 40:2257-67, 2002; Guttman, et al., Parkinsonism Relat Disord. 7:231-234,2001). Some preliminary results indicate that treatment efficacy for apathy in association with Alzheimer's disease can exist with acetylcholinesterase inhibitors (Cummings, et al., Am J Geriatr Pharmacother., 3: 137-48, 2005; Wynn, et al. Dement Geriatr Cogn Disord. 17:100-8, 2004; Boyle, et al., Dement Geriatr Cogn Disord. 17:91-9, 2004). Other preliminary results indicate treatment efficacy for apathy in schizophrenia can exist with atypical antipsychotics (Sumiyoshi T, et al., Int J Neuropsychopharmacol. 8:451-5, 2005; Goldstein, Drugs Today (Bare), 35:193-210 (1999); Tollefson, et al., Am J Psychiatry. 54: 466-74,1997).

B. Tacke et al. in Pharmakopsychiat (1975) 7: 82-89 describes the use of piracetam in the treatment of schizophrenic patients, o.a. for alleviating apathy. A. Trevisio et al. in Minerva Pediatrica (1977) 29: 1267-1272 discloses the use of piracetam in children. WO03/018005 discloses the use of nefiracetam in the treatment of neurodegeneration after stroke. In Int J Geratr Psychiatr (2002) 17: 1099-1105 apathy is described as a symptom of Parkinson's disease. K. Okada et al. in Stroke (1997) 28: 2437-2441 discloses the examination of patients for apathy after stroke. M Glenn at al., in Brain Injury (2002) 16: 509-516 discloses the examination of patients with traumatic brain injury for apathy. Life Science (2003) 74: 281-291 discloses the effect of nefiracetam on certain receptors which are downregulated in Alzheimer patients. JP 9087178A discloses the use of aniracetam in the treatment of neurodenerative diseases including Alzheimer's disease.

There is thus a need for an effective treatment for apathy syndrome in human subjects.

### References

Joy S, Kaplan E, Fein D. Arch Clin Neuropsychol. 2004; 19(6):759-67. Speed and memory in the WAIS-III Digit Symbol-Coding subtest across the adult lifespan.
Joy S, Kaplan E, Fein D. Clin Neuropsychol. 2003; 17(2):182-94. Digit Symbol-Incidental Learning in the WAIS-III: construct validity and clinical significance.
Marin RS. Apathy: a neuropsychiatric syndrome. J Neuropsychiatry Clin Neurosci. 1991; 3(3):243-54.
Marin RS, Biedrzycki RC, Firinciogullari S. Reliability and validity of the Apathy Evaluation Scale. Psychiatry Res. 1991; 38(2):143-62.
Landes AM, Sperry SD, Strauss ME, Galdmacher DS. Apathy in Alzheimer's disease. J Am Geriatr Soc. 2001; 49(12):1700-7.
Starkstein SE, Fedoroff JP, Price TR, Leiguarda R, Robinson RG. Apathy following cerebrovascular lesions. Stroke. 1993; 24(11):1625-30.
Van Reekum, R. et al., Apathy: why care? J. Neuropsychi. Clin Neurosci.,17 (1): 7-19,2005.
Andersson S, Krogstad JM, Finset A. Apathy and depressed mood in acquired brain damage: relationship to lesion localization and psychophysiological reactivity. Psychol Med. 1999;29(2):447-56.
Marin RS, Firinciogullari S, Biedrzycki RC. Group differences in the relationship between apathy and depression.
J Nerv Ment Dis. 1994;182(4):235-9.
Duffy J. Apathy in neurologic disorders. Curr Psychiatry Rep. 2000; 2(5):434-9.

### SUMMARY OF THE INVENTION

The invention provides a method of treating apathy syndrome in a human subject. In one embodiment, the subject suffers from conditions associated with or characterized by depression. The subject is first evaluated to determine whether one or more behavioral characteristics of apathy are present. If such characteristics are observed, the subject is administered a pharmaceutical composition comprising nefiracetam, in an amount effective to produce an improvement in such characteristics.

The effective amount of nefuacetam is in the range of about 300 to about 1800 mg/day for an adult subject, preferably in the range of about 600 mg to about 1200 mg/day, the administering can be by oral administration, and can be carried out over an extended period of at least several days to several weeks or several months.

Evaluating the human subject to determine whether the subject shows one or more behavioral characteristics associated with apathy can be carried out through application of the diagnostic criteria for apathy and by determining the subject's score on the Apathy Scale or Apathy Evaluation Scale. The method can further include periodically testing the subject to determine to subject's score on the Apathy Scale or Apathy Evaluation Scale, and adjusting the dose, if necessary to achieve a desired degree of improvement in such score.

The method can also be effective to improve frontal or executive cognitive functions in the subject, as evidenced, for example, by an improvement in the subject's score on the symbol digit modality test (SDMT).

These and other objects and features of the invention will become more fully apparent from the following detailed description of the invention.

### DETAILED DESCRIPTION OF THE INVENTION

### Definitions

When present, unless otherwise specified, alkyl groups are from 1 to 12 carbon atoms, either straight chained or branched, preferably from 1 to 8 carbon atoms, and more preferably 1 to 6 carbon atoms.

Apathy syndrome is a clinical syndrome primarily due to lack of motivation, especially in comparison with the patient's previous level of function or standards appropriate for their age and culture. In addition, apathy syndrome is further characterized by a diminution in goal-directed behavior (for example, reduced effort or an increased dependency on others to structure activity) and in goal-directed cognition (for example, reduced interest in learning new things or a loss of concern about one's personal problems) as well as in the concomitants of goal-directed behavior (for example, a lack of emotional responsively to positive or negative events). This clinical state is a source of distress and is associated with impairment in social, occupational or other important areas of functioning. Apathy syndrome is not attributable to emotional distress, intellectual impairment, a diminished level of consciousness or to the direct effects of a substance (for example, a medication or a drug of abuse). Terms which are related to apathy, or which may be synonymous with apathy, include abulia (sometimes reflecting severe apathy) and amotivational states. Apathy syndrome, as used herein, often, but not always, involves frontal-subcortical system dysfunction (van Reekum, et al., J Neuropsychiatry ClinNeurosci. 17:7-19,2005; Marin., J Neuropsychiatry Clin Neurosci. 3:243-54, 1991).

Apathy Evaluation Scale (AES) is used as a measure of apathy severity. Items and factor structure of AES are listed in Marin, et al. (Psychiatry Res. 1991; 38(2):143-62). The assessment, the impairment, as well as and the need of recovery of apathy and cognitive function after stroke are illustrated by Robert G. Robinson "The Clinical Neuropsychiatry of Stroke", 1998, Cambridge University Press, pages 143, 222-225 and 292-293.

Apathy Scale (AS) is an abbreviated version of the AES. Items and scoring of Papthy Scale can be found, for example, at Starkstein, et al., J. Neuropsychiatry Clin. Neurosciences, 4: 134-139 (1992).

Frontal-subcortical dysfunction means signs and symptoms associated with a loss or impairment of normal neuronal activity in frontal brain regions and in their connections to structures beneath the frontal cortex including the thalamus, basal ganglia and amygdala.

A CNS disorder associated with or characterized by frontal-subcortical dysfunction means a disorder that injures, destroys or impairs the activity of neuronal systems largely projecting within and between the frontal lobe and subcortical structures including the amygdala, basal ganglia, and thalamus.

Cognitive function can be measured using a tool called the symbol digit modality test (SDMT). In this test, patients have a legend which links each digit to a specific graphical, non-text symbol. The patients have a pre-specified, fixed period of time to match, in writing, as many digits to a list of symbols as possible. The number of correctly matched digits to symbols provides a measure of a range of cognitive functions, including executive function, attention, memory, and motor function (Joy, et.al. Arch Clin Neuropsychol. 19:759-67, 2004; Joy, et.al. Clin Neuropsychol. 17:182-94; 2003)

Nefiracetam refers to compound having the chemical structure (N-(2,6-dimethylphenyl)-2-(2-oxo-1-pyrrolidinyl) acetamide), and includes derivatives of nefiracetam having minor chemical-group substitutions, e.g., on the pyrrolidine or phenyl rings, that do not significantly alter the physicochemical properties of the compound or its activity. For example, the pyrrolidine ring can be substituted at positions 3, 4 or 5 with a methyl or other small radical group, e.g., methyl or OH group, or the phenyl ring can be substituted at its 2 and/or 6 positions to replace the methyl groups with a hydrogen atom or other small radical group, or substituted at one or more of its 3, 4, or 4 positions.

Pharmaceutically acceptable salts are salts that retain the desired biological activity of the parent compound and do not impart undesired toxicological effects. Pharmaceutically acceptable salt forms include various polymorphs as well as the amorphous form of the different salts derived from acid or base additions. The acid addition salts can be formed with inorganic or organic acids. Illustrative but not restrictive examples of such acids include hydrochloric, hydrobromic, sulfuric, phosphoric, citric, acetic, propionic, benzoic, napthoic, oxalic, succinic, maleic, malic, adipic, lactic, tartaric, salicylic, methanesulfonic, 2-hydroxyethanesulfonic, toluenesulfonic, benzenesulfonic, camphorsulfonic, and ethanesulfonic acids. The pharmaceutically acceptable base addition salts can be formed with metal or organic counterions and include, but are not limited to, alkali metal salts such as sodium or potassium; alkaline earth metal salts such as magnesium or calcium; and ammonium or tetraalkyl ammonium salts, i.e., NX₄⁺ (wherein X is C₁₋₄).

Applicants have discovered a method of treating apathy syndrome of any cause in a human subject. The method comprises: (a) evaluating a subject to determine whether the subject shows one or more behavioral characteristics of apathy, and (b) if the subject shows one or more said characteristics, administering to the subject nefiracetam or a pharmaceutically acceptable salt thereof, in an amount effective to produce an improvement in said characteristics,

In one embodiment, the present methods treats apathy syndrome in a human subject suffering from conditions associated with or characterized by frontal-subcortical dysfunction, for example, a CNS disorder. The present methods treats apathy syndrome in a human subject suffering from depression. Such patients can be identified by standard diagnostic tests for this condition.

The method involves first evaluating the subject to determine whether one or more behavioral characteristics of apathy are present. This evaluation is typically done using the standard Apathy Evaluation Scale or the more abbreviated Apathy Scale, and identifying apathy syndrome by the score achieved by the subject in one or more of the apathy criteria. Subjects scoring above normal on one or more of the apathy criteria are identified as candidates for treatment.

The compound is administered to the human subject in an amount effective to produce an improvement in characteristics associated with apathy, and in particular, in those subject characteristics identified by the initial evaluation. Thus, after a period of several days to several weeks or several months of treatment, the subject can be reevaluated, e.g., using the AES or Apathy Scale, to determine whether one or more of the original characteristics of apathy has been reduced, as evidenced by a lower apathy score. Depending on the degree of improvement, the compound dose can be changed, e.g., increased, until a desired improvement in apathy score is observed. According to another feature of the invention, the treated subject can also show an improvement in cognitive function, as measured, for example, by the symbol digit modality test (SDMT) described above.

The compound can be administered to the human subject in the form of tablets, capsules, suppositories, syrups, aqueous or oily suspensions, gels, dispersible powders, granules, or emulsion. Oral composition of the compound can be prepared in a manner well known per se in the pharmaceutical art and usually include the Formula (I) compound, e.g., nefiracetam, as an active ingredient, in admixture or otherwise in association with a pharmaceutically acceptable carrier or diluent thereof For making those formulations said active ingredient will usually be mixed with a carrier, or diluted with a diluent, or enclosed or encapsulated in a capsule, sachet, cachet, paper or other suitable container. Suitable carriers and diluents are known per se.

The amount of nefiracetam, administered in oral form will vary and can be any effective amount according to the physician's prescription. Normally, depending upon the patient and the mode of administration, the quantity of compound administered orally can vary over a wide range to provide from about 1 mg/kg to about 25 mg/kg, usually 5 mg/kg to 20 mg/kg of body weight of the patient per dose. Unit doses of nefiracetam, in the oral pharmaceutical compositions can contain, for example, from about 50 mg to about 1800 mg for an adult subject, usually from 600 to 1200 mg of the compound and can be administered 1 to 4 times daily, usually 2 times daily. These dose levels are adjusted accordingly for smaller patients or children. The treatment is typically continued over a period of at least several days to several weeks or several months, and depending on the underlying brain disorder, can be continued over the lifetime of the subject.

The compound of the present invention can be administered by any of the accepted modes of systemic administration including oral, parenteral, intravenous, intramuscular, and subcutaneous, transdermal, transmucosal, and rectal; with oral administration being preferred.

Any pharmaceutically acceptable mode of administration can be used, including solid, semi-solid, or liquid dosage forms, such as, tablets, suppositories, pills, capsules, powders, granules, liquids suspensions, injections, or the like, preferably in unit dosage form suitable to single administration ofprecise dosages, or in sustained or controlled release forms for prolonged administration of the compound at a predetermined rate. The compositions typically include a conventional pharmaceutical carrier or excipient and the active compound and, in addition, can include other medicinal agents, pharmaceutical agents, carriers, etc. Many examples of such pharmaceutically acceptable vehicles can be found in Remington's Pharmaceutical Sciences (17th edition (1985)) and other standard texts. These preparations can be prepared by any conventional methods.

The carriers useful for these preparations include all organic or inorganic carrier materials that are usually used for the pharmaceutical preparations and are inert to the active ingredient. Examples of the carriers suitable for the preparation of tablets capsules, granules and fine granules are diluents such as lactose, starch, sucrose, D-mannitol, calcium sulfate, or microcrystalline cellulose; disintegrators such as sodium carboxymethylcellulose, modified starch, or calcium carboxymethylcellulose; binders such as methylcellulose, gelatin, acacia, ethylcellulose, hydroxypropylcellulose, or polyvinylpyrrolidone; lubricants such as light anhydrous silicic acid, magnesium stearate, talc, or hydrogenated oil; or the like. When formed into tablets, they can be coated in a conventional manner by using the conventional coating agents such as calcium phosphate, carnauba wax, hydroxypropyl methylcellulose, macrogol, hydroxypropyl methylphthalate, cellulose acetate phthalate, titanium dioxide, sorbitan fatty acid ester, or the like.

Examples of carriers suitable for the preparation of syrups are sweetening agents such as sucrose, glucose, fructose, or D-sorbitol; suspending agents such as acacia, tragacanth, sodium carboxymethylcellulose, methylcellulose, sodium alginate, microcrystalline cellulose, or veegum; dispersing agents such as sorbitan fatty acid ester, sodium lauryl sulfate, or polysorbate 80; or the like. When formed into syrups, the conventional flavoring agents, aromatic substances, preservatives, or the like can optionally be added thereto. The syrups can be in the form of dry syrup that is dissolved or suspended before use.

Examples of carriers used for the preparation of suppositories are cocoa butter, glycerin saturated fatty acid ester, glycerogelatin, macrogol, or the like. When formed into suppositories, the conventional surface active agents, preservatives or the like can optionally be admixed.

When formed into injections, the compound is dissolved in a suitable solvent for injection, to which can optionally be added the conventional solubilizers, buffering or pH adjusting agents, isotonic agents, preservatives and other suitable substances. The injections can be in the solid dry preparations, which are dissolved before use.

For solid compositions, conventional non-toxic carriers include, for example mannitol, lactose, starch, magnesium stearate, sodium saccharin, talcum, cellulose, glucose, sucrose, magnesium carbonate, and the like can be used. The active compound as defined above can be formulated as suppositories using, for example, polyalkylene glycols such as propylene glycol as a carrier. Liquid pharmaceutical compositions can, for example, be prepared by dissolving, dispersing, etc. an active compound as defined above and optional pharmaceutical adjuvants in a carrier to form a solution or suspension. If desired, the pharmaceutical composition can also contain minor amounts of non-toxic auxiliary pH buffering agents and the like, for example, sodium acetate, sorbitan monolaurate, triethanolamine oleate, etc. Actual methods of preparing such dosage forms are known, or will be apparent to those skilled in this art; for example, see Remington's Pharmaceutical Sciences, Mack Publishing Company, Easton, Pa., 15th Edition, 1975. The composition or formulation to be administered will, in any event, contain a quantity of the active compound(s) in an amount effective to alleviate the symptoms of the subject being treated.

Dosage forms or compositions contain active ingredient of Formula (I) compound in the range of 0.25 to 95% with the balance made up from non-toxic carrier can be prepared. For oral administration, a pharmaceutically acceptable non-toxic composition is formed by the incorporation of any of the normally employed recipients, and can contain 1%-95% active compound(s), preferably 5%-50%.

Parenteral administration is generally characterized by injection, whether subcutaneously, intramuscularly, or perineurally. Injectables can be prepared in conventional forms, either as liquid solutions, suspensions, or emulsions. In addition, the pharmaceutical compositions can also contain minor amounts of non-toxic substances such as wetting or emulsifying agents, auxiliary pH buffering agents and the like, such as, sodium acetate, sorbitan monolaurate, triethanolamine oleate, etc.

The percentage of active compound contained in such parenteral compositions is dependent on the specific nature thereof, as well as the activity of the compound and the needs of the subject.

For delayed release, the compound can be formulated in a pharmaceutical composition, such as in microcapsules formed from biocompatible polymers, nanomilled active compound, or in liposomal carrier systems according to methods known in the art.

For continuous release of active agent, the compound can be covalently conjugated to a water soluble polymer, such as a polylactide or biodegradable hydrogel derived from an amphipathic block copolymer, as described in U.S. Patent No. 5,320,840. Collagen-based matrix implants, such as described in U.S. Patent No. 5,024,841, are also useful for sustained delivery of therapeutics.

The following examples further illustrate the present invention. These examples are intended merely to be illustrative of the present invention and are not to be construed as being limiting.

### EXAMPLES having some degree of depression

### Example 1. Treatment of Apathy in Post-Stroke Patients

The activity of nefiracetam to improve apathy and/or cognitive function in post-stroke patients has been demonstrated by a controlled clinical trial against placebo. In this trial, nefiracetam was administered orally to 103 post-stroke patients, who all have some degree of depression, for up to twelve weeks after the vascular event. Concurrently, 56 patients received placebo (placebo group). Of the 103 post-stroke patients treated with nefiracetam, 55 received 600 mg daily (600 mg group), and 48 received 900 mg daily (900 mg group). The three groups of patients were followed for up to 12 weeks, and evaluated at the end of week 4, the end of week 8, and the end of week 12, by measuring the Apathy Evaluation Scale (a symptom scale for measuring apathy), and by measuring SDMT (for measuring cognitive function).

There was a dose-dependent and time-dependent change in apathy syndrome for the nefiracetam-treated patients in comparison to the placebo group, where larger numeric changes mean larger improvements in apathy. As of the last visit (12 weeks), the 900 mg group showed an average improvement of 3.4 points, the 600 mg group showed an average improvement of 2.1 points, and the placebo group showed an average improvement of 1.4 points. In comparison, at 4 weeks, the 900 mg group showed only a 1.9 point improvement in apathy, the 600 mg group showed only a 1.9 point improvement, and the placebo an average improvement of 1.4 points.

The change in apathy was also more marked for patients who were treated within 28 days of the stroke, where the 900 mg group showed an average improvement of 6.1 points, the 600 mg group showed an average improvement of 1.3 points, and the placebo group showed an average improvement of 0.6 points. Nefiracetam worked particularly well in improving apathy for patients who had suffered cortical strokes. At the last visit carried forward, the 900 mg group of cortical stroke patients showed an average improvement of 5.2 points, compared with 3.4 for the 600 mg group and 1.0 for placebo. Last visit carried forward is a standard method of data analysis whereby patient drop-outs are dealt with by using the value obtained at the patient's last actual visit as the value for all subsequently scheduled, but missed, visits when no data could be collected.

When patients with cortical strokes and significant baseline apathy (greater than 18) were studied, the improvement in apathy in the 900 mg group was 11.7 points at the last visit, compared with 9.4 points in the 600 mg group and 5.4 points in the placebo group.

Nefiracetam also showed dose-dependent and time-dependent improvements in cognitive function as measured by the SDMT. As of the last visit, the 900 mg group showed an average improvement of 6.6 points, the 600 mg group showed an average improvement of 5.7 points, and the placebo group showed an average improvement of 4.2 points. In comparison, at 4 weeks, the 900 mg group showed only a 4.4 point change, the 600 mg group showed only a 4.1 point change, and the placebo group showed only a 3.2 point change. The improvements in cognitive function were more marked for patients with cortical strokes. The improvement seen in the SDMT scale in the 900 mg group for patients with cortical strokes was 14.2 points at week 12, in comparison with 7 points in the 600 mg group at week 12, and 2.7 points for the placebo group at week 12.

The clinical findings thus show that in the treatment of post-stroke patients, the treatment should be initiated as early as possible, preferably within the first few months, and preferably as soon as possible, e.g., within the first month following the stroke event.

### Example 2. Treatment of Apathy in Depression Patients

In 151 post-stroke patients having moderate to severe depression, the intervention-free data of Apathy Scale (AS), as a valid measure of apathy, and of Hamilton Rating Scale of Depression (HAM-D) and Beck Depression Inventory (BDI), as valid measures of depression, were collected and analyzed using the method of confirmatory factor analysis. HAM-D and BDI are fully validated and are widely used measures of the severity of depression. HAM-D (Hamilton, J. Neurol. Neurosurg. Psychiatry. 23:56-62, 1960) is rated by a professional. BDI (Arch Gen Psychiatry. 4:561-571,1961) is rated by a patient.

The 151 patients, who were evaluated at least once after treatment, included the 103 patients studied in Example 1. The 151 patients were divided in three groups each orally receiving placebo (51 patients) or nefiracetam at 600 mg/day (54 patients) or nefiracetam at 900 mg/day (46 patients).

The data at the end of 12 weeks were analyzed and summarized as follows:
(a) As time went, patients' apathy measured by the Apathy Scale declined from pretreatment for patients on active treatments as well as for patients on placebo; however, patients on active treatment, especially on the high dose medication, showed more reduction in apathy than did patients on placebo. This finding is consistent across patients with moderate to severe depression and patients with severe depression, and the treatment effect appears to have occurred early for more depressed patients;
(b) As time went, patients' apathy measured by the Apathy Scale declined from pretreatment for patients on active treatments as well as for patients on placebo; however, patients on active treatment, especially on the high dose medication, showed more reduction in apathy than did patients on placebo. This finding is consistent across patients with various severity of baseline apathy, and the treatment effect appears to have been slightly large for more severe patients.
(c) As time went, patients' depression measured by HAM-D declined from pretreatment for patients on active treatments as well as for patients on placebo; however, unlike the treatment effects observed on patients' apathy, patients on placebo showed slightly more reduction in depression than did patients on active treatment. This finding is consistent across patients with various severity of baseline apathy, and the placebo effect appears to have been slightly larger than the treatment effect, especially for patients on the lower active dose. The results indicate apathy and depression are distinguishable entities.

Although the invention has been described with respect to particular embodiments and applications, it will be appreciated how various changes and modifications can be made without departing from the invention.

## Claims

1. A pharmaceutical composition comprising nefiracetam or a pharmaceutically acceptable salt thereof for use in treating apathy syndrome in a human subject suffering from depression.

2. The composition for use according to Claim 1, wherein said human subject suffers from conditions associated with or **characterized by** frontal-subcortical dysfunction.

3. The composition for use according to Claim 1, wherein the effective amount of nefiracetam is in the range of 300 to 1800 mg/day for an adult subject.

4. The composition for use according to Claim 3, wherein said effective amount of nefiracetam is in the range of 600 mg to 1200 mg/day for an adult subject.

5. The composition for use according to Claim 1, where said composition is for administration over an extended period of at least several weeks.

6. The composition for use according to Claim 1, wherein said composition is for oral administration.

7. The composition for use according to Claim 1, wherein the subject suffering from depression is evaluated to determine whether the subject shows one or more behavioral characteristics of apathy.

8. The composition for use according to Claim 1, wherein the apathy syndrome in said subject is evaluated by determining the subject's score on the Apathy Scale or Apathy Evaluation Scale.

9. The composition for use according to Claim 8, wherein the evaluation of the apathy syndrome further comprises the steps of periodically testing the subject to determine to subject's score on Apathy Scale or Apathy Evaluation Scale, and adjusting the effective amount of nefiracetam, if necessary, until an improvement in said score is observed.

10. The composition for use according to Claim 1, which is effective on improving cognitive function in the subject.

11. The composition for use according to Claim 10, wherein the subject's score on the symbol digit modality test is improved.

## Patentansprüche

1. Pharmazeutische Zusammensetzung, die Nefiracetam oder ein pharmazeutisch annehmbares Salz davon umfasst, zur Verwendung bei der Behandlung des Apathiesyndroms bei einer humane Person, der an einer Depression leidet.

2. Zusammensetzung zur Verwendung gemäß Anspruch 1, wobei die humane Person an Zuständen leidet, die mit einer frontal-subkortikalen Dysfunktion verbunden oder **dadurch gekennzeichnet** sind.

3. Zusammensetzung zur Verwendung gemäß Anspruch 1, wobei die wirksame Menge des Nefiracetams im Bereich von 300 bis 1800 mg/Tag für eine erwachsene Person liegt.

4. Zusammensetzung zur Verwendung gemäß Anspruch 3, wobei die wirksame Menge des Nefiracetams im Bereich von 600 bis 1200 mg/Tag für eine erwachsene Person liegt.

5. Zusammensetzung zur Verwendung gemäß Anspruch 1, wobei die Zusammensetzung zur Verabreichung über einen längeren Zeitraum von wenigstens mehreren Wochen bestimmt ist.

6. Zusammensetzung zur Verwendung gemäß Anspruch 1, wobei die Zusammensetzung zur oralen Verabreichung bestimmt ist.

7. Zusammensetzung zur Verwendung gemäß Anspruch 1, wobei die Person, die an einer Depression leidet, bewertet wird, um zu bestimmen, ob die Person ein oder mehrere Verhaltensmerkmale der Apathie zeigt.

8. Zusammensetzung zur Verwendung gemäß Anspruch 1, wobei das Apathiesyndrom bei der Person bewertet wird, indem man den Score der Person auf der Apathieskala oder Apathiebewertungsskala bestimmt.

9. Zusammensetzung zur Verwendung gemäß Anspruch 8, wobei die Bewertung des Apathiesyndroms weiterhin die Schritte des regelmäßigen Testens der Person, um den Score der Person auf der Apathieskala oder Apathiebewertungsskala zu bestimmen, und gegebenenfalls des Einstellens der wirksamen Menge an Nefiracetam, bis eine Verbesserung in dem Score beobachtet wird, umfasst.

10. Zusammensetzung zur Verwendung gemäß Anspruch 1, die die kognitive Funktion bei der Person wirksam verbessert.

11. Zusammensetzung zur Verwendung gemäß Anspruch 10, wobei der Score der Person im Symbol-Digit-Modality-Test verbessert ist.

## Revendications

1. Composition pharmaceutique comprenant du néfiracétam ou un de ses sels pharmaceutiquement acceptables, à utiliser dans le traitement du syndrome apathique chez un sujet humain souffrant de dépression.

2. Composition à utiliser selon la revendication 1, où ledit sujet humain souffre de troubles associés à, ou **caractérisés par**, un dysfonctionnement frontal-sous-cortical.

3. Composition à utiliser selon la revendication 1, où la quantité efficace de néfiracétam se situe dans le domaine de 300 à 1800 mg/jour pour un sujet adulte.

4. Composition à utiliser selon la revendication 3, où la quantité efficace de néfiracétam se situe dans le domaine de 600 mg à 1200 mg/jour pour un sujet adulte.

5. Composition à utiliser selon la revendication 1, ladite composition étant destinée à une administration sur une période prolongée d'au moins plusieurs semaines.

6. Composition à utiliser selon la revendication 1, ladite composition étant destinée à une administration orale.

7. Composition à utiliser selon la revendication 1, où on évalue le sujet souffrant de dépression pour déterminer si le sujet présente une ou plusieurs caractéristiques comportementales de l'apathie.

8. Composition à utiliser selon la revendication 1, où on évalue le syndrome d'apathie chez ledit sujet en déterminant le score du sujet sur l'échelle de l'apathie ou l'échelle d'évaluation de l'apathie.

9. Composition à utiliser selon la revendication 8, où l'évaluation du syndrome d'apathie comprend en outre les étapes de test périodique du sujet pour déterminer le score du sujet sur l'échelle de l'apathie ou l'échelle d'évaluation de l'apathie, et d'ajustement de la quantité efficace de néfiracétam, si nécessaire, jusqu'à ce que l'on observe une amélioration dudit score.

10. Composition à utiliser selon la revendication 1, qui est efficace pour améliorer la fonction cognitive chez le sujet.

11. Composition à utiliser selon la revendication 10, où le score du sujet dans le test SDMT *(Symbol Digit Modalities Test)* est amélioré.
